Europäisches Patentamt

European Patent Office

Office européen des brevets

(19)

(11) Veröffentlichungsnummer: **0 272 706 B1**

(12) **EUROPÄISCHE PATENTSCHRIFT**

(45) Veröffentlichungstag der Patentschrift: **28.10.92**

(21) Anmeldenummer: **87201320.6**

(22) Anmeldetag: **13.07.87**

Die Akte enthält technische Angaben, die nach dem Eingang der Anmeldung eingereicht wurden und die nicht in dieser Patentschrift enthalten sind.

(51) Int. Cl.⁵: **A61K 31/425**, //(A61K31/425, 31:275)

(54) **Verfahren zur Herstellung eines veterinären Heilmittels, sowie Verarbeitung in pharmazeutisch anwendbare Formen.**

(30) Priorität: **11.12.86 NL 8603150**

(43) Veröffentlichungstag der Anmeldung:
**29.06.88 Patentblatt 88/26**

(45) Bekanntmachung des Hinweises auf die Patenterteilung:
**28.10.92 Patentblatt 92/44**

(84) Benannte Vertragsstaaten:
**BE DE ES FR GB GR LU NL**

(56) Entgegenhaltungen:
**AU-B- 527 154**

**DIALOG 04229235, Medline 66-89, August; K.I.ALTAIF: "Effect of anthelmintic treatment on the performance of Awassi sheep in Iraq"**

(73) Patentinhaber: **Hak, Barend Willem
Giessensesteeg 12 A
Giessen(NL)**

(72) Erfinder: **Hak, Barend Willem
Giessensesteeg 12 A
Giessen(NL)**

(74) Vertreter: **Siemens, Andreas Meinhard Ernest, Dipl.-Ing.
SIEMENS & CIE. Roskam 8
NL-4813 GZ Breda(NL)**

**Beschreibung**

Die vorliegende Erfindung bezieht sich auf die Herstellung eines veterinären Heilmittels gegen Parasiten bei Vieh, sowie auf die Verarbeitung in pharmazeutische Form.

In Anbetracht der vielen verschiedenen Parasitenarten bei Vieh bedarf es eines präventiven und therapeutischen Präparates, das sowohl oral als auch parenteral angewendet werden kann zur Bekämpfung von Endo- und Ectoparasiten bei Rindern, Schafen, Ziegen und Schweinen.

Ein derartiges Präparat, welches stabil ist, einfach an zu wenden ist und eine Breitspektrumaktivität besitzt, war noch nicht bekannt.

Weil Infektion mit Ectoparasiten durch die Haut oft das Auftreten von Endoparasiten zur Folge hat, und der Zeitpunkt einer erforderlichen Therapie bei manchen Tieren schwer zu erkennen ist, ergibt ein Präparat mit solchen Eigenschaften und Anwendungsmöglichkeiten einen Ausweg für die Veterinärpraxis.

Blutsaugende Rundwürmer (Nematoden) stellen eine ernste Bedrohung des Viehbestandes dar, jedoch mit den bekannten Mitteln dagegen konnten die gleichfalls auftretenden Larven von verschiedenen Arthropoden nicht gleichzeitig bekämpft werden.

Es war insbesondere schwierig, Trematoden und Arthropodenlarven neben Nematoden zu bekämpfen bezw. eine vorbeugende Behandlung gegen mehrere Gruppen dieser Parasiten zu ermöglichen.

Das Präparat, welches gemäss der vorliegenden Erfindung hergestellt wird, erfüllt die genannten Kriterien, und es ist sowohl zur Vorbeugung als auch zur Therapie bei Kühen, Schafen, Ziegen und Schweinen bei Infektionen durch erwachsene Wurmarten und durch Larvenstadien von Wurmarten und von Arthropoden einsetzbar.

Viele Parasiten haben ihre Entwicklung in verschiedenen Gasttieren, wobei ihr Metabolismus grosse Anpassungsmöglichkeiten zeigt.

Es ist deshalb ein Fortschritt, dass mit dem vorliegenden Präparat Darmnematoden, Lungenwurmspezies, Leberegel sowie die Larven verschiedener gefährlicher und irritierender Arthropoden beweisbar gut bekämpft werden konnten.

Nicht nur der Erreger der Distomatose (Leberegelkrankheit), welcher als Larve in der Leberegelschnecke auftritt, sich auf dem Grase encystiert und dann mit dem Gras in Rind und Schaf gelangt, wo der Saugwurm dann in die Gallengänge vordringt und Leberschädigungen verursacht, doch auch Toxacara vitulorum, Trichuris ovis, Trichostrongyliden, Haemonchus, Nematodirus und Strongyloides sowie die Bremsenarten Oestrus ovis, Hypoderma bovis und Hypoderma lincatum, Dermatobia hominis, Boophilus decoloratus, Chorioptes bovis und Psoroptes bovis bei Rindern, Cochliomya hominivorax bei Schafen und Metastrongylus elongatus, Ascaris und die Larven derselben, Hyostrongylus rubidus, Oesophagostomum dentatum und verschiedene andere konnten erfolgreich bekämpft werden.

Das erfindungsgemässe veterinärmedizinische Präparat gegen Parasiten bei Vieh, das sowohl zur oralen, subcutanen und parenteralen Anwendung zur Therapie und zur Vorbeugung geeignet ist, besteht aus einem stabilen Gemisch der Komponenten 2,3,5,6-tetrahydro-6-phenyl-imidazo-2,1-thiazolhydrochlorid (Formel I) und N-[5-Chlor-4-$\alpha$-Chlorphenyl-$\alpha$-Cyanmethyl-2-methylphenyl]-2-hydroxy-3,5-dijodbenzamid (Formel II), mit einigen Lösungs- und Stabilisierungsstoffen, die zur Stabilisierung des pH-wertes des flüssigen Suspensionsgemisches dienen.

Das Phenylimidazothiazolderivat ist reines $\ell$-Isomeres von Tétramisol als Hydrochlorid und es wird bereitet durch Reaktion und Annellierung von ortho-Phenylendiamin mit ortho-Thiopropionsäure-amid durch Erhitzen unter Rückflusskühlung.

Die Bruttoformel ist $C_{11}H_{12}N_2S.HC\ell$; das Molekulargewicht der reinen Verbindung ist 240,8.

Die zweite Komponente, das Dijodbenzamidderivat, ist ein Salicylanilid.

Es wird bereitet durch Reduktion von 2-Nitro-4-Chlortoluol mit Chlorbenzyl-4-cyanid in alkalischem Medium, Reduktion des Reaktionsproduktes zu 4-amino-2-Chlor-$\alpha$-4-Chlorphenyl-5-methylphenyl-acetonitril, welches mit Phosphortrichlorid in Phosphat umgesetzt wird, das mit 3,5-Dijodsalicylsäure reagiert zu N-[5-Chlor-4-$\alpha$-Chlorphenyl-$\alpha$-cyanmethl-2-methylphenyl]-2-hydroxy-3,5-dijodbenzamid. Formel: $C_{22}H_{14}C\ell_2J_2N_2O_2$. Mol.gewicht: 663.08. Die Herstellung des erfindungsgemässen Präparates findet wie folgt statt:

In einen Kessel aus rostfreiem Stahl mit Rührwerk von etwa 300 l Inhalt werden 100 kg Propylenglykol gebracht und unter fortwährendem Rühren werden nacheinander zugesetzt:

| 250 g | Methyl-4-hydroxybenzoat |
|---|---|
| 25 g | Propyl-4-hydroxybenzoat |
| 12500 g | Polyvinylpyrrolidon |
| 25000 g | 2,3,5,6-Tetrahydro-6-phenyl-imidazo-2,1-thiazol-hydrochlorid (laevo-Tetramisol) |
| 100000 g | Wasser pro inject. |
| 2500 g | Zitronensäure |
| 250 g | Natriumdisulfit. |

Dann fügt man soviel Natriumcitrat zu als Pufferung, dass der pH-Wert der Lösung genau 3.0 beträgt.

Die erhaltene Lösung wird nun sterilisiert und filtriert über ein Membranfilter mit einer Poriengrösse von < 0.22 μm.

Unter weiterem fortwährendem Rühren wird zur Lösung gegeben:

| 12500 g | N-[5-Chlor-4-(4-Chlorphenyl)-Cyanomethyl-2-methylphenyl]-2-hydroxy-3,5-dijodbenzamid. |
|---|---|
| 1250 g | Kolloidales Kieselsäuregel. |

Das Rühren wird während 30 min. intensiv fortgesetzt, sodass eine homogene Suspension erhalten wird.

Die Konzentration des Präparates in flüssiger Form wird erhalten, indem Wasser pro inject. zugefügt wird, sodass 1 l des flüssigen Präparates 100 g l-Tetramisol und 50 g des Dijodbenzamid-derivats enthält.

Das flüssige Präparat wird sodann abgefüllt in Flaschen aus braunem Glas bezw. in Ampullen von 100 ml Inhalt aus braunem Glas.

Es muss vor Licht geschützt gelagert werden und ist dann einige Jahre haltbar.

Es ist für orale und subkutane Verabreichung geeignet.

Die Suspension kann aber auch mit einem inerten salbenbildenden Träger vermischt werden und in einer Form als Kreme oder Salbe auf die blossgestellten Haut- oder Fellteile gebracht werden.

Eine dritte Art der Anwendungsform für das erfindungsgemässe Präparat ist gegeben, indem die Suspension mit einem festen inerten pulverförmigen Trägermaterial in geeigneter Konzentration verrieben und die erhaltene Masse zu Tabletten, Zäpfchen und Bolus gepresst wird.

Die Zusammensetzung der Suspension wird analytisch geprüft und soll vorzugsweise wie folgt lauten: 1L der Suspension enthält:

| 100.0 g | l-Tetramisol |
|---|---|
| 50.0 g | Dijodbenzamidderivat |
| 1.0 g | Methyl-4-hydroxybenzoat |
| 0.1 g | Propyl-4-hydroxybenzoat |
| 1.0 g | Natriumdisulfit |
| 50.0 g | Polyvinylpyrrolodon |
| 100.0 g | Zitronensäure (mit Natriumcitrat gepuffert, sodass der pH-Wert der Lösung 3.0 beträgt). |
| 400.0 g | Propylenglykol |
| 5.0 g | Kolloide Kieselsäure. |

Aufgefüllt mit demin. steril. Wasser auf 1 l.

Das Präparat ist eine weisse wenig-viskose wässerige homogene Suspension mit einem Spez. Gewicht 1,1. Analyse der beiden aktiven Komponenten kann nebeneinander stattfinden in einem Hochdruck-Flüssigkeitschromatographen mit einer Säulenpackung von Nucleosil RP 18 (Teilchen 10 μm).

Die Detektion des 1-Tetramisols in methanolischer Lösung zeigt sich mit UV bei 225 nm.

Die Detektion des Dijodbenzamidderivats gelöst in Acetonitril findet sich mit Fluoreszenz-Spektrometrie als Excitation bei 336 nm und als Emission bei 506 nm.

Die Haltbarkeit des Präparats und die Stabilität der Konzentrationen der aktiven Komponenten konnten in Versuchen mit einer Fehlergrenze von ±0.6% nachgewiesen werden.

Das Dijodbenzamidderivat kann ausserdem analytisch bestimmt werden mittels potentiometrischer Titration in nicht-wässrigem Bad, indem 0.3 g des Präparates in 60 ml N,N-Dimethylformamid gelöst wurde und titriert wurde mit 0.1-molarer Tetrabutyl-ammoniumhydroxydlösung p.a.

Ein Verbrauch von 1 ml 0.1 mol. Tetrabutylammoniumhydroxyd kommt überein mit 0.06631 g N-[5-Chlor-4-(4-Chlorphenl)- Cyanomethyl-2-methyphenyl]-2-hydroxy-3,5-dijodbenzamid.

Es wurden akute, subakute und subchronische Toxizitätsversuche an Warmblütern oral und subkutan ausgeführt, wobei sich ergab, dass die $LD_{50}$ subkutan mehr als 40 mg/kg Körpergewicht betrug.

Die Kombination der beiden aktiven Komponenten gemäss der vorliegenden Erfindung zeigte jedoch bei Nematoden, Trematoden und anderen schmarotzenden Würmern sowie bei Larven von Gliederfüsslern (Arthropoden) eine schnelle paralysierende Wirkung infolge von Blockierung von Adenosintriphosphat, wodurch der Metabolismus des Parasiten nicht mehr funktioniert und der Parasit abgetötet und ausgeschieden wird.

Säugetiere unterliegen keinerlei Schädigung,weil im Blut und Verdauungstrakt ein inaktiver Metabolit gebildet wird.

Das Präparat zeigte sich bei Versuchen auch sehr wirksam gegen Haemonchus-Formen,die resistent waren gegen Benzimidazolpräparate bekannter Zusammensetzung.

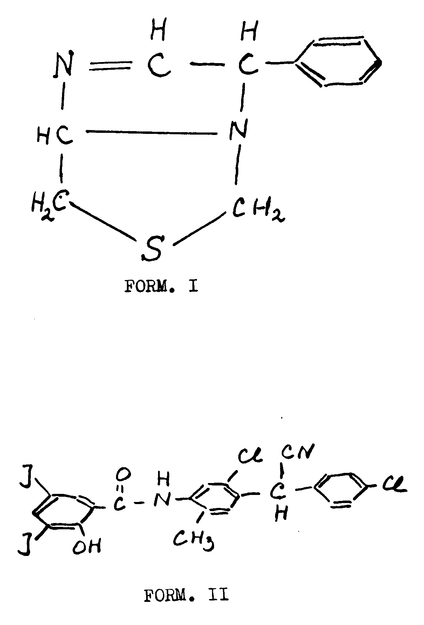

**Patentansprüche**

1. Verfahren zur Herstellung eines veterinärmedizinischen Präparates zur oralen und parenteralen therapeutischen und präventiven bekämpfung von Endo- und Ectoparasiten bei Rindern, Schafen, Ziegen und Schweinen, dadurch gekennzeichnet, dass man eine Suspension von reinem 2,3,5,6-tetrahydro-6-phenyl-imidazo-2,1-thiazol-hydrochlorid (l-Tetramisol) und von N-[5-Chlor-4-$\alpha$-Chlorphenyl-$\alpha$-Cyanmethyl-2-Metylphenyl]-2-hydroxy-3,5-dijodbenzamid im Gewichtsverhältnis 2:1 in Propylenglykol unter Zusatz geringerer Mengen von Methyl-4-Hydroxybenzoat, Propyl-4-Hydroxybenzoat, Natriumdisulfit, Polyvinylpyrrolidon, Zitronensäure mit Natriumcitratpuffer,und kolloider Kieselsäure herstellt und das stabile Gemisch in eine geeignete pharmazeutische Anwendungsform bringt.

2. Veterinärmedizinisches Präparat, hergestellt nach Anspruch 1, dadurch gekennzeichnet, dass es in flüssiger Form 10 gew•/vol.% reines l-Tetramisol und 5 gew•/vol.% N-[5-Chlor-4-$\alpha$-Chlorphenyl-$\alpha$-Cyanmethyl-2-Methylphenyl]-2-hydroxy-3,5-Dijodbenzamid mit stabilisierenden Hilfsstoffen in Propylenglykol enthält und in verabreichbare Form gebracht ist.

3. Veterinärmedizinisches Präparat zur Bekämpfung von Parasiten auf und im Körper von Vieh, dadurch gekennzeichnet, dass es nebeneinander l-2,3,5,6-tetrahydro-6-phenyl-imidazo-2,1-thiazolhydrochlorid und N-[5-Chlor-4-$\alpha$-Chlorphenyl-ᴖ-Cyanmethyl-2-Methylphenyl]-2-Hydroxy-3,5-Dijodbenzamid enthält.

4. Bolus zur antiparasitären Behandlung von Vieh, dadurch gekennzeichnet, dass darin ein Präparat nach Anspruch 3 enthalten ist.

5. Kapsel zur antiparasitären Behandlung von Vieh, dadurch gekennzeichnet, dass darin ein Präparat nach Anspruch 3 enthalten ist.

6. Injektionsflüssigkeit zur antiparasitären Behandlung von Vieh, dadurch gekennzeichnet, dass darin ein Präparat nach Anspruch 3 enthalten ist.

7. Pharmazeutische Anwendungsformen des Präparates nach Anspruch 2, dadurch gekennzeichnet, dass sie in Fläschchen aus braunem Glas abgefüllt werden.

**Claims**

1. A method for the preparation of a veterinary remedy for the oral and parenteral therapeutic and preventive fight against endo- and ectoparasites with cattle, sheep, goats and swine, characterized in that a suspension of pure 2,3,5,6-tetrahydro-6-phenyl-imidazo-2,1-thiazole-hydrochloride (l-tetramisol) and of N-[5-chloro-4-$\alpha$-chloro-phenyl-$\alpha$-cyanomethyl-2-methylphenyl]-2-hydroxy-3,5-dijodobenzamide in a weight ratio of 2:1 in propylene-glycol is made with addition of small amounts of methyl-4-hydroxybenzoate, propyl-4-hydroxybenzoate, sodiumdisulphite, polyvinylpyrrolidone, citric acid with buffer of sodium citrate, and colloidal silicic acid, and that this stable mixture is worked up into an appropriate form for pharmaceutical administration.

2. A veterinary remedy made according to claim 1, characterized in that as a liquid it comprises 10% wt./vol. of pure l-tetra-misol and 5% wt./vol. of N-[5-chloro-4-$\alpha$-chlorophenyl-$\alpha$-cyanomethyl-2-methyl-

phenyl]-2-hydroxy-3,5-dijodobenzamide with stabilizing additives in propylene glycol,and that it is worked up in a form suitable for administration.

3. A veterinary remedy for fight against parasites upon and inside the bodies of cattle, characterized in that it comprises a composition of l-2,3,5,6-tetrahydro-6-phenyl-imidazo-2,1-thiazole-hydrochloride and N-[5-chloro-4-α-chlorophenyl-α-cyanomety-2-metylphenyl]-2-hydroxy-3,5-dijodobenzamide.

4. A bolus for antiparasitical treatment of cattle, characterized in that said bolus comprises a composition according to claim 3.

5. A capsule for antiparasitical treatment of cattle, characterized in that said capsule comprises a composition according to claim 3.

6. A liquid for injection for antiparasitic treatment of cattle, characterized in that said liquid comprises a composition according to claim 3.

7. Pharmaceutically applicable items of the preparation according to claim 2, characterized in that they are bottled in small vials of brown glass.

**Revendications**

1. Une méthode de préparation d'un remède vétérinaire pour le traitement thérapeutique et préventif , contre les endo- et les ectoparasites de bétail, des moutons, des chèvres et des porcs, d'une manière orale ou parenterale, caractérisée en ce que l'on prépare une suspension du 2,3,5,6-tetrahydro-6-phenyle-imidazo-2,1-thiazole-hydrochloride (l-tetramisol) et du N-[5-chlore-4-α-chlorophenyle-α-cyanométhyle-2-méthylephenyle]-2-hydroxy-3,5-dijodobenzamide,en proportions de rapport en poids de 2:1, dans le propylèneglycol, en ajoutant de moindres quantités de methyle-4-hydroxybenzoate, de propyle-4-hydroxy-benzoate, de disulphite de sodium, de polyvinyl-pyrrolidone, d'acide citrique avec citrate de sodium comme tampon, et d'acide silicique colloidale, et que ladite mélange stable est traitée en forme appropriée pour l'administration pharmaceutique.

2. Un remède vétérinaire préparé d'après la revendication 1, caractérisé en ce qu'il contient en forme liquide de 10% poids/volume de l-tetramisol et de 5% poids/volume de N-[5-chlore-4-α-chlorophenyle-α-cyanométhyle-2-methyle-phenyle]-2-hydroxy-3,5-dijodobenzamide,avec additifs stabilisants en propylène-glycol, et qu'il est traité en forme utilisable pour le dosage.

3. Un remède vétérinaire pour le traitement contre les parasites sur la peau et dans les corps du bétail, caractérisé en ce qu'il comprend une composition de l-2,3,5,6-tetrahydro-6-phényle-imidazo-2,1-thiazole-hydrochloride et de N-[5-chlore-4-α-chlorophényle-α-cyanométhyle-2-méthyxphényle]-2-hydroxy-3,5-dijodobenzamide.

4. Une boule pour le traitement antiparasitique du bétail, caractérisé en ce que ladite boule comprend une composition selon la revendication 3.

5. Une capsule pour le traitement antiparasitique du bétail, caractérisé en ce que ladite capsule comprend une composition selon la revendication 3.

6. Un liquide à l'injection pour le traitement antiparasitique du bétail, caractérisé en ce que ledit liquide comprend une composition selon la revendication 3.

7. Objets pharmaceutiques à l'application du rémède d'après la revendication 2, caractérisés en ce qu'ils sont embouteillés dans petites fioles de verre brun.

FORM. I

FORM. II